# EUROPÄISCHE PATENTSCHRIFT

(11) **EP 0 946 206 B1**
(45) Veröffentlichungstag und Bekanntmachung des Hinweises auf die Patenterteilung: **20.03.2002**
(21) Anmeldenummer: 97953910.3
(22) Anmeldetag: 23.12.1997
(51) Int. Cl.: A61L 2/10

(54) **VORRICHTUNG MIT MINDESTENS EINER OBERFLÄCHENSCHICHT AUS HALBLEITERMATERIAL**
DEVICE WITH AT LEAST ONE SEMICONDUCTOR SURFACE LAYER
DISPOSITIF COMPORTANT AU MOINS UNE COUCHE SUPERFICIELLE SEMICONDUCTRICE

(30) Priorität: 23.12.1996 DE 19654109
(43) Veröffentlichungstag der Anmeldung: 06.10.1999
(73) Patentinhaber: Massholder, Karl F., Dr., 69250 Schönau (DE)
(72) Erfinder: Massholder, Karl F., Dr., 69250 Schönau (DE)
(74) Vertreter: Isenbruck, Günter, Dr.
(86) Internationale Anmeldenummer: EP9707252
(87) Internationale Veröffentlichungsnummer: WO9828012

(56) Entgegenhaltungen:
- EP-A- 0 714 041
- DATABASE WPI Section PQ, Week 9648 Derwent Publications Ltd., London, GB; Class P41, AN 96-480395 XP002069934 & JP 08 243 434 A (EBARA CORP)
- OTGER NEUFANG: "GRUNDLAGEN DER OPTOELEKTRONIK", 1982, AT-VERLAG, AARAU/SCHWEIZ

## Beschreibung

Die Erfindung betrifft eine Vorrichtung nach Anspruch 1 mit mindestens einer Oberflächenschicht mit einer desinfizierbaren und/oder oxidierenden Außenseite. Die Erfindung betrifft weiterhin ein Verfahren nach Anspruch 11 zum Desinfizieren und/oder Oxidieren auf der Außenseite einer Oberflächenschicht.

In vielen Bereichen des täglichen Lebens spielt Hygiene eine wichtige Rolle. Dies betrifft insbesondere den Gesundheitsbereich. So müssen beispielsweise in Krankenhäusern oder Arztpraxen bestimmte Bereiche oder Gerätschaften, wie Operationstische, möglichst keimarm oder sogar keimfrei gehalten werden. Auch beim Umgang mit Lebensmitteln, die für den menschlichen Verzehr bestimmt sind, muß man eine erhöhte Sorgfalt im Hinblick auf Hygiene walten lassen. Hierzu gehören beispielsweise Verkaufstheken für Lebensmittel, Arbeitsflächen in Betrieben, die Lebensmittel verarbeiten, wie Metzgereien oder Gastwirtschaften, aber auch Molkereien mit ihren Getränkeleitungen. Neben einer Vielzahl von weiteren Anwendungsfällen sollen auch noch die Forschung, Diagnostik und Applikation in den Bereichen Mikrobiologie und Biotechnologie genannt werden.

In all diesen Bereichen ist es wünschenswert oder sogar notwendig, daß man bestimmte Flächen keimarm oder sogar keimfrei hält. Das gleiche gilt für innere Oberflächen von Behältern, Rohrleitungen und Ventilen.

Es ist hierzu bekannt, offene Flächen durch mechanische Reinigung mit bakteriziden Mitteln sauber zu halten. Gerätschaften kann man gegebenenfalls unter Anwendung von höheren Temperaturen sterilisieren. In manchen Fällen verwendet man auch eine Bestrahlung mit UV-Licht. Diese ist allerdings nicht immer voll wirksam und auch im Hinblick auf die Gefährdung oder Belastung von Menschen vielfach problematisch, deshalb sind großflächige UV-Desinfektionen nur in arbeitsfreien Zeiträumen, z.B. über Nacht, im Einsatz.

Der Nachteil bei den bekannten Reinigungsverfahren ist, daß die Verkeimung unmittelbar nach dem Reinigungsvorgang wieder einsetzen kann. Die bakterizide Wirkung hängt von der Sorgfalt der jeweiligen Reinigungskraft und den Reinigungsintervallen ab. Ein hoch keimfreier Zustand ist daher nur sehr umständlich und mit hohem Aufwand aufrechtzuerhalten. Erschwerend kommt hinzu, daß einige Bakterien eine gewisse Resistenz gegen die bakteriziden Mitteln entwickeln, so daß eine Entkeimung trotz sorgfältigen Arbeitens tatsächlich nicht oder nicht in ausreichendem Maße stattfindet. Dieser Fehler wird unter Umständen oft nicht einmal bemerkt.

Noch schwieriger ist die Reinigung innerer Oberflächen, die nur gespült oder mit Hilfe von Dampf entkeimt werden können. Beim Spülen gibt es immer wieder Toträume und "kalte" Stellen, die von der Reinigung nicht erfaßt werden. Hierbei können sich dann Bakterienkolonien bilden, die eine nachfolgende Verkeimung begünstigen. Außerdem muß für eine Reinigung der Betrieb eingestellt werden. Das gleiche gilt auch für äußere Oberflächen. Großflächige Desinfektionsmaßnahmen mit Formaldehyd oder Ethylenoxid stellen aus toxikologischer Sicht große Probleme dar, da diese Gase in das Material eindringen und relativ lange Ausgaszeiten haben (z. B. Quarantänestationen). Hierdurch muß ein größerer Vorrat von Instrumenten oder Räumen bereit gehalten werden, da die Verfügbarkeit durch diese Zeiten begrenzt ist.

Aus JP-A-8-243434 ist eine Vorrichtung zur Reinigung von Räumen bekannt, mit einer UV-Strahlungsquelle, einem Fotoelektronen-Emitter, Elektroden zur Erzeugung eines elektrischen Feldes und einem Sammler zum Auffangen der geladenen Teilchen, wobei der Fotoelektronen-Emitter aus einem mit einem Fotoelektronen-Emissionsmaterial überzogenen gläsemen Werkstoff besteht. Hierbei wird der äußere Fotoeffekt ausgenutzt, d.h. mittels UV-Strahlen geeigneter Energie werden aus dem Fotoelektronen-Emissionsmaterial Fotoelektronen nach außen abgegeben; in dem zu reinigenden Raum befindliche Teilchen werden dadurch aufgeladen und mittels eines Sammlers aufgefangen.

Der Erfindung liegt die Aufgabe zugrunde, eine Vorrichtung mit mindestens einer Oberflächenschicht mit einer Außenseite anzugeben, die leichter und mit höherer Zuverlässigkeit desinfiziert werden kann und/oder oxidierend wirkt.

Weiterhin ist es Aufgabe der Erfindung, eine Vorrichtung anzugeben, die es ermöglicht, innere Oberflächen zu desinfizieren, die durch direkte Einstrahlung von UV-Licht nicht erreicht werden können. Ferner ist es Aufgabe der Erfindung, ein Verfahren zum Desinfizieren und/oder Reinigen auf der Außenseite einer Oberflächenschicht zur Verfügung zu stellen.

Die Lösung geht aus von einer Vorrichtung mit mindestens einer Oberflächenschicht aus einem Halbleitermaterial mit einer Innenseite, die auf einem Träger aufliegt und einer desinfizierbaren und/oder oxidierenden Außenseite sowie mit einer UV-Strahlungsquelle.

Die Vorrichtung weist einen Träger auf, der lichtleitend ist und aus der UV-Strahlungsquelle wird UV-Strahlung über den lichtleitenden Träger unmittelbar auf die Innenseite des Halbleitermaterials eingekoppelt, wobei die Außenseite der Oberflächenschicht desinfiziert wird und/oder oxidierend wirkt, wobei die Vorrichtung keinen Sammler zum Auffangen von Fotoelektronen aufweist.

Lichtleiter sind Anordnungen von optischen Komponenten zum Weiterführen von Licht durch Totalreflexion, wobei insbesondere die Abnahme des Brechungsindex innerhalb des Lichtleiters von innen nach außen eine große Rolle spielt. Für einen guten Lichtleiter ist es erstrebenswert, die Lichtverluste durch Absorption, Streuung und Abstrahlung möglichst gering zu halten. Es wurde nun überraschenderweise gefunden, daß die geringe, bislang unerwünschte Strahlungsabgabe nach außen entlang eines Lichtwellenleiters ausreichend ist, die Innenseite eines darauf aufgebrachten Halbleiters zu aktivieren und somit die Desinfektion und/oder Oxidation zu bewirken.

Man kann nun die Gegenstände oder Arbeitsflächen, deren Reinigung oder Desinfektion man wünscht, mit dem lichtleitenden Träger und der Oberflächenschicht aus einem Halbleitermaterial versehen. Es ist jedoch auch möglich, die zu desinfizierenden oder zu reinigenden Gegenstände selbst als lichtleitende Träger auszubilden und darauf die Oberflächenschicht aus dem Haltleitermaterial aufzubringen.

Um die nachfolgende Erläuterung zu vereinfachen, wird im folgenden immer von "Desinfektion" die Rede sein, auch wenn es sich nur um eine Verringerung der Keimzahl an der Oberfläche handelt. Eine vollständige Entkeimung ist zwar vielfach möglich, aber nicht immer erforderlich oder gewünscht.

Um die Oberflächenschicht zu desinfizieren, wird die UV-Lichtquelle in Betrieb gesetzt, so daß sie UV-Strahlung abgibt. Diese UV-Strahlung wird allerdings gegen die von außen nicht sichtbare Innen- oder Rückseite des Halbmaterials gerichtet. Die Oberfläche kann also durchaus mit Arbeitsgerätschaften oder anderen Gegenständen, die bei einer Bestrahlung der Außenseite zu einer Abschattung führen könnten, bedeckt sein. Die UV-Strahlung muß nun nicht mehr nach außen austreten, um zu der gewünschten Desinfektion zu führen. Obwohl die biologischen, physikalischen und chemischen Abläufe hierbei noch nicht völlig geklärt sind, nimmt man an, daß die UV-Strahlung das Halbleitermaterial anregt, also fotoaktiviert. Fotoaktivierung bedeutet, daß durch die Lichtabsorption im Halbleiter, z.B. n-TiO₂, Elektronen vom Valenzband in das Leitungsband gehoben werden. Hierdurch entsteht ein Redoxpotential, das über die Bildung radikalischer Spezies bzw. Mechanismen zur Abtötung von Mikroorganismen führt. Da diese Prozesse unspezifisch sind, kommt es nebenbei auch zu oxidativen Abbaureaktionen. Da sich der Halbleiter nicht verändert spricht man von einem Katalysator. Die so von innen bestrahlte Oberfläche wird also auf einfache Art und Weise desinfiziert. Eine derartige Desinfektion kann auch während der normalen Benutzung erfolgen, so daß eine Keimfreiheit oder zumindest Keimarmut auch während des Betriebs aufrechterhalten werden kann.

Zwischen der UV-Lichtquelle und der Innenseite ist ein lichtleitender Träger angeordnet. Damit wird man freier im Hinblick auf die Anordnung der UV-Lichtquelle in Bezug zu der Oberflächenschicht. In manchen Fällen ist es nicht möglich, die Lichtquelle an der Rück- oder Innenseite der Oberflächenschicht anzuordnen, weil dort kein Raum zur Verfügung steht. Wenn man nun einen lichtleitenden Träger verwendet, kann man die UV-Strahlung auch von anderen Orten an die Innenseite des Halbleitermaterials führen.

Der lichtleitende Träger kann in bevorzugter Weise als Platte ausgestaltet sein, beispielsweise als Tischplatte eines Operationstisches.

Der plattenförmige Träger ist dann bevorzugt einseitig, im Bedarfsfall aber auch beidseitig mit Halbleitermaterial beschichtet.

Es ist jedoch auch möglich, den lichtleitenden Träger rohrförmig auszubilden, so daß Rohrleitungen als Meterware, einschließlich der Verbindungsstücke, zur Verfügung gestellt werden. Im Falle rohrförmig ausgestalteter Träger ist bevorzugt die Rohrinnenfläche mit dem Halbleitermaterial beschichtet, es besteht jedoch auch die Möglichkeit, rohrförmige Träger auf der Rohraußenfläche mit dem Halbleitermaterial zu beschichten.

Der lichtleitende Träger kann weiterhin als Behälter ausgebildet sein, dessen Innenfläche mit dem Halbleitermaterial beschichtet ist. Es ist jedoch auch möglich, die Innenfläche eines Behälters aus einem beliebigem Material mit dem lichtleitenden Träger und anschließend mit dem Halbleitermaterial zu versehen. Als Behälter mit desinfizierbarer Innenfläche kommen beispielsweise Lagertanks in Frage.

Der lichtleitende Träger kann starr sein, es ist jedoch möglich, verformbare, biegsame, flexible Materialien als lichtleitende Träger einzusetzen. Flexible, schlauchförmige Träger mit bevorzugt auf der Außenfläche aufgebrachtem Halbleitermaterial führen zu in der Medizin verwendeten Kathetern. Weiterhin können als flexible lichtleitende Trägermaterialien Fasern, Gewebe oder Vliese zum Einsatz kommen.

Das Trägermaterial kann bevorzugt Quarz sein, oder aber auch ein lichtleitendes Polymermaterial. Als lichtleitende Polymermaterialien eignen sich beispielsweise Fluorpolymere, aber auch Polymethylmethacrytat.

Lichtleitende Polymermaterialien können sehr flexibel ausgebildet werden und sind gleichzeitig relativ unempfindlich gegen mechanische Belastungen. 'Man kann also auch Oberflächen mit einer Oberflächenschicht versehen, die nicht eben ausgebildet sind. Darüber hinaus gibt es eine Reihe von Polymeren, die UV-Licht durchlassen.

Das Halbleitermaterial wird bevorzugt in einer dünnen Schicht auf das lichtleitende Trägermaterial aufgetragen. Bei einer Bestrahlung mit UV-Licht läßt das lichtleitende Trägermaterial die Strahlung passieren und von der Innenseite her auf das Halbleitermaterial treffen. Das Trägermaterial nimmt also neben der Aufgabe des Haltens des Halbleiters auch die Aufgabe der Lichtleitung war. Damit kann man die Oberflächenschicht relativ dünn machen.

Vorzugsweise reflektiert das Trägermaterial zumindest auf der dem Halbleitermaterial gegenüberliegenden Seite zum Halbleitermaterial hin. Man kann eine derartige Ausgestaltung vorteilhafterweise dazu ausnutzen, die UV-Strahlung von der Seite her in das Trägermaterial einzuleiten. Da eine derartige Strahlung immer einen gewissen Strahlungswinkel hat, die Strahlung also grob gesagt einen Strahlungskegel bildet, wird ein Teil der UV-Strahlung direkt auf die Innenseite des Halbleitermaterials treffen, während ein anderer Teil der Strahlung auf den "Boden" des Trägermaterials trifft. Von dort wird die Strahlung aber wieder in Richtung auf das Halbleitermaterial reflektiert, so daß man auch größere Breiten oder Längen mit einer seitlich oder stirnseitig angeordneten UV-Lichtquelle versorgen kann.

Vorzugsweise ist das Halbleitermaterial als dünne Schicht auf dem Trägermaterial ausgebildet. Das Halbleitermaterial deckt also das Trägermaterial bevorzugt vollflächig ab. Damit besteht nicht die Gefahr, daß die UV-Strahlung nach außen tritt. Die Desinfektion kann also unabhängig davon vorgenommen werden, ob Personen in der Nähe sind oder nicht.

Hierbei ist besonders bevorzugt, daß das Halbleitermaterial eine Dicke in der Größenordnung von wenigen Molekülen aufweist. Hierbei führt die Anregung der "untersten" Moleküle der Schicht des Halbleitermaterials, d.h. der Moleküle, die unmittelbar vom UV-Licht beaufschlagt werden, recht schnell zu einer entsprechenden Aktivierung der oberen Moleküle der Halbleiterschicht, die dann zu einer Inaktivierung der Keime oder Bakterien führt.

Vorzugsweise ist das Halbleitermaterial durch Titandioxid oder Siliziumcarbid gebildet. Diese Materialien stehen preisgünstig zur Verfügung. Sie haben sich bei der Desinfektion von Oberflächen, die mit einer derartigen Oberflächenschicht versehen sind, bewährt.

Vorzugsweise weist die UV-Strahlung eine Wellenlänge im Bereich von 280 bis 400 nm, insbesondere im Bereich von 320 bis 380 nm auf.

Normalerweise ist die desinfizierende Wirkung von UV-Strahlung nur bei einer Wellenlänge von ca. 254 nm gegeben. Diese ist aber für den Menschen wegen ihrer mutagenen Wirkung auf Hautzellen und besonders schädigenden Wirkung auf die Augen gefährlich. Die beanspruchte Wellenlänge im Bereich von 280 bis 400 nm, insbesondere 320 bis 380 nm (UVA) ist jedoch ungefährlich. Derartiges UV-Licht wird sogar für kosmetische und therapeutische Zwecke eingesetzt. Besonders dann, wenn man einen Wellenlängenbereich von 350 nm oder höher wählt, hat die Verwendung einer derartigen Wellenlänge den Vorteil, daß man das UV-Licht nicht sieht, insbesondere nicht davon geblendet wird.

In einer bevorzugten Ausgestaltung ist vorgesehen, daß im Halbleitermaterial und/oder in der UV-Lichtquelle und/oder im lichtleitenden Trtäger ein fluoreszierender Farbstoff angeordnet ist. Dieser Fluoreszenzfarbstoff kann beispielsweise in einem Polymer eingebettet sein. Dieses Polymer kann das transparente Trägermaterial oder einen anderen Lichtleiter bilden.

Durch Einlagerung des Fluoreszenzfarbstoffes kann man visuell kontrollieren, ob die UV-Lichtquelle in Betrieb ist oder nicht. Darüber hinaus hat diese Ausgestaltung den Vorteil, daß UV-Licht dieser Wellenlänge von formbaren Polymeren geleitet werden kann. Die bislang für die Desinfektion verwendete UV-Strahlung von ca. 254 nm konnte nur über Leiter aus Quarz geleitet werden.

Zudem wirkt der Fluoreszenzfarbstoff sozusagen als Lichttransformator. Er setzt UV-Strahlung, die von der UV-Lichtquelle in einem größeren Spektrum abgegeben wird, um in eine von dem jeweiligen Farbstoff bestimmte Strahlung mit einem relativ eng umgrenzten Wellenlängenbereich. Damit kann man die Energie der UV-Lichtquelle noch besser ausnutzen.

Zusätzlich zu der UV-Lichtquelle kann man auch eine Anregungseinrichtung verwenden, die das Halbleitermaterial eiektrisch anregt. Auch bei einer elektrischen Anregung kann man freie Valenzen erzeugen, d.h. Elektronen vom Valenzband in das Leitungsband anheben, was über das oben angesprochene Redoxpotential wiederum die Keime inaktiviert.

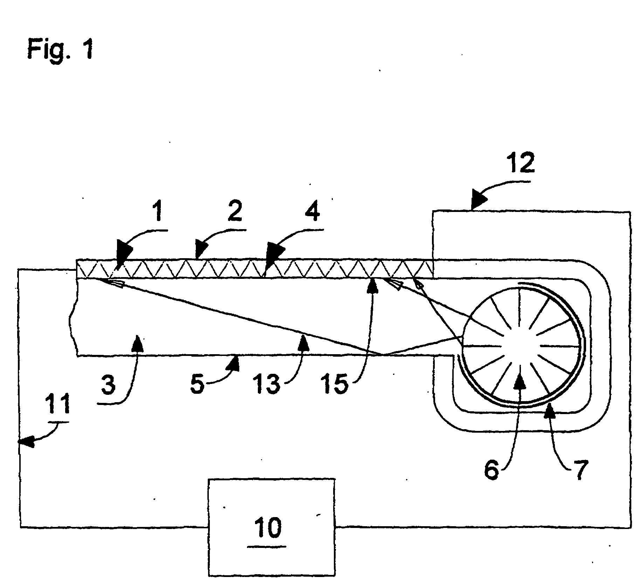

Die Erfindung wird im folgenden anhand eines bevorzugten Ausführungsbeispiels in Verbindung mit der Zeichnung beschrieben. Hierin zeigt
- die einzige Figur: einen schematischen Querschnitt durch eine erfindungsgemäße Vorrichtung.

Die Oberflächenschicht 1 aus einem Halbleitermaterial 4 liegt mit der Innenseite 15 auf einem lichtleitenden Träger auf. Die Außenseite der Oberflächenschicht bildet dann die desinfizierbare und/oder oxidierende Fläche. Die Oberflächenschicht mit desinfizierbarer und/oder oxidierende Außenseite und der lichtleitende Träger, auf dem die Oberflächenschicht mit der Innenseite aufliegt, können auf einer nicht näher dargestellten Gerätschaft, beispielsweise einem Arbeitstisch, oder Operationstisch auf der Innenwand eines Behälters oder der Innen- oder Außenwand eines Rohres aufgebracht werden. Es ist jedoch auch möglich, eine Gerätschaft selbst, beispielsweise einen Operationstisch, Rohrleitungen oder Katheter als lichtleitende Träger auszubilden, auf denen ein Halbleitermaterial aufliegt. Das lichtleitende, UV-transparente Trägermaterial kann beispielsweise durch ein lichtleitendes Polymermaterial gebildet sein. Derartige Polymere haben den Vorteil, daß sie auch nachträglich auf Oberflächen aufgebracht werden können.

Als Halbleitermaterial 4, das insbesondere als dünne Schicht, bevorzugt mit einer Schichtdicke in der Größenordnung von wenigen Molekülen, aufgebracht wird, kommt beispielsweise Titandioxid (TiO₂) oder Siliciumcarbid in Betracht.

An der der Außenseite 2 gegenüberliegenden Seite 5, die der Einfachheit halber auch als Unterseite bezeichnet wird, ist das Trägermaterial 3 reflektierend ausgebildet, d.h. die Unterseite 5 reflektiert Licht, das aus Richtung der Außenseite 2 einstrahlt, wieder zur Außenseite 2 zurück.

Diese Reflexionseigenschaften sind insbesondere im Zusammenhang mit UV-Strahlung von Bedeutung. Hierzu weist die Vorrichtung eine UV-Lichtquelle 6 auf, die UV-Strahtung mit einer Wellenlänge im Bereich von 280 bis 400 nm, insbesondere im Bereich von 320 bis 380 nm abgibt. Die UV-Lichtquelle ist mit einem Reflektor 7 versehen, der die von der UV-Lichtquelle 6 abgegebene UV-Strahlung seitlich in das transparente und lichtleitende Trägermaterial 3 hinein richtet. Wie anhand der Pfeile 13 zu erkennen ist, trifft ein Teil der Strahlung direkt auf die Innenseite der Schicht 4 aus Halbleitermaterial. Ein anderer Teil der UV-Strahlung wird an der Unterseite 5 des Trägermaterials 3 in Richtung auf die Schicht aus Halbleitermaterial reflektiert.

Wenn an der dargestellten Position für die UV-Lichtquelle 6 kein Raum zur Verfügung stehen sollte, dann kann man die UV-Strahlung auch über geeignete Lichtleitfasern bis zu der entsprechenden Einleitstelle in das Trägermaterial führen.

In nicht dargestellter Weise kann zwischen der Schicht 4 des Halbleitermaterials und der UV-Lichtquelle 6 auch noch ein Fluoreszenzfarbstoff angeordnet sein, beispielsweise in dem Trägermaterial 3 oder in einem anderen Lichtleiter. Dieser fluoreszierende Farbstoff dient als "Lichttransformator", d.h. er wird von der UV-Strahlung der Lichtquelle 6 angeregt, auch wenn diese Strahlung einen relativ großen Spektralbereich des UV-Lichts enthält. Er gibt dann aber UV-Licht mit einem relativ kleinen Spektralbereich oder sogar nur mit einer einzigen Wellenlänge ab. Wenn nun diese Wellenlänge oder der kleine Spektralbereich auf das Halbleitermaterial gut abgestimmt ist, dann kann man eine ausgezeichnete Ausnutzung der Energie der Lichtquelle 6 erreichen.

Das Halbleitermaterial ist zwar nur als dünne Schicht 4 auf das Trägermaterial 3 aufgetragen. Diese Schicht reicht aber aus, um einen Austritt der UV-Strahlung zu verhindern oder zumindest soweit abzuschwächen, daß ein Betrachter keinen unangenehmen Eindruck erhält oder sogar belästigt oder gefährdet wird. Die Schicht 4 ist nur wenige Moleküle stark. Wenn nun UV-Strahlung aus der Lichtquelle 6 auf die Innenseite der Schicht 4 trifft, dann wird dort der Halbleiter, beispielsweise TiO₂ angeregt. Man nimmt an, daß dann ein Elektron nach "innen" wandert, d.h. auf ein anderes Energieniveau gehoben wird. Hierbei entsteht dann eine freie Valenz, die, wenn ein Keim an dieser Stelle liegt, diesen oxidiert und damit inaktiviert. Da die Halbleiterschicht 4 das Trägermaterial flächig bedeckt und die UV-Strahlung von der UV-Lichtquelle 6 die gesamte Schicht 4 von innen her beaufschlagt, wird dementsprechend jeder Keim, der sich an der Oberfläche aufhält oxidiert und damit inaktiviert.

Zusätzlich zur Anregung des Halbleitermaterials in der Schicht 4 durch UV-Strahlung, die von der Rückseite her auftrifft, kann man auch eine elektrische Anregung verwenden. Hierzu ist eine elektrische Anregungseinrichtung 10 vorgesehen, die über zwei Elektroden 11, 12 mit der Schicht 4 verbunden ist. Mit einer elektrischen Anregung kann man die Anregung des Halbleitermaterials durch die UV-Strahlung unterstützen.

Die Verwendung der auf einem Träger 3 aufliegenden Oberflächenschicht 1 ist nicht auf ebene Flächen beschränkt. Man kann also nicht nur Arbeitsflächen, Tische, Wände, Böden oder Außenseite von irgendwelchen Arbeitsgeräten damit versehen oder daraus ausbilden. Man kann eine derartige Oberflächenschicht beispielsweise auch an der Innenwand von Rohren oder von beliebig geformten Gegenständen anbringen. Dies kann sogar nachträglich erfolgen, indem man beispielsweise lichtleitende Polymere in bestehende Rohrleitungen einbringt, wie dies bereits jetzt zur Eindämmung von Korrosion geschieht. Die Schicht 4 kann permanent auf das Trägermaterial 3 aufgebracht werden.

## Patentansprüche

1. Vorrichtung mit mindestens einer Oberflächenschicht (1) mit einer Innenseite (15), die auf einem lichtleitenden Träger (3) aufliegt und mit einer Außenseite (2) sowie mit einer UV-Strahlungsquelle (6), wobei die Oberflächenschicht (1) aus einem Halbleitermaterial (4) gebildet ist, aus der UV-Strahlungsquelle (6) Uv-Strahlung (13) über den lichtleitenden Träger (3) unmittelbar auf die Innenseite (15) des Halbleitermaterials (4) eingekoppelt wird, und dabei die Außenseite (2) der Oberflächenschicht (1) desinfiziert wird und/oder oxidierend wirkt, wobei die Vorrichtung keinen Sammler zum Auffangen von Fotoelektronen aufweist.

2. Vorrichtung nach Anspruch 1, **dadurch gekennzeichnet, daß** der Träger (3) als Platte ausgebildet ist, die einseitig oder beidseitig mit dem Halbleitermaterial (4) beschichtet ist.

3. Vorrichtung nach Anspruch 1, **dadurch gekennzeichnet, daß** der Träger (3) rohrförmig ausgebildet ist und das Halbleitermaterial (4) auf der Rohrinnenfläche und/oder auf der Rohraußenfläche aufgebracht ist

4. Vorrichtung nach Anspruch 1, **dadurch gekennzeichnet, daß** der Träger (3) als Behälter ausgebildet ist, dessen Innenfläche mit dem Halbleitermaterial (4) beschichtet ist.

5. Vorrichtung nach einem der Ansprüche 1 bis 4, **dadurch gekennzeichnet, daß** das Trägermaterial Quarz oder ein lichtleitendes Polymermaterial, insbesondere Polymethylmethacrylat, ist.

6. Vorrichtung nach einem der Ansprüche 1 bis 5, **dadurch gekennzeichnet, daß** der Träger plastisch oder elastisch verformbar ist.

7. Vorrichtung nach einem der Ansprüche 1 bis 6, **dadurch gekennzeichnet, daß** das Halbleitermaterial (4) als dünne Schicht, insbesondere mit einer Schichtdicke in der Größenordnung von wenigen Molekülen, ausgebildet ist.

8. Vorrichtung nach einem der Ansprüche 1 bis 7, **dadurch gekennzeichnet, daß** das Halbleitermaterial (4) Titandioxid oder Siliciumcarbid ist.

9. Vorrichtung nach einem der Ansprüche 1 bis 8, **dadurch gekennzeichnet, daß** im Halbleitermaterial (4) und/oder in der UV-Lichtquelle (6) und/oder im lichtleitenden Träger (3) ein fluoreszierender Farbstoff angeordnet ist.

10. Vorrichtung nach einem der Ansprüche 1 bis 9, **dadurch gekennzeichnet, daß** zusätzlich zur UV-Lichtquelle (6) eine Anregungseinrichtung (10) vorgesehen ist, die das Halbleitermaterial (4) elektrisch anregt.

11. Verfahren zum Desinfizieren und/oder Oxidieren auf der Außenseite einer Oberflächenschicht (1) aus einem Halbleitermaterial (4), die mit der Innenseite (15), auf einem Träger (3) aufliegt, unter Verwendung einer UV-Strahlungsquelle (6), **dadurch gekennzeichnet, daß** UV-Strahlung (13) aus der UV-Strahlungsquelle (6) durch Lichtleitung über den Träger (3) unmittelbar auf die Innenseite (15) des Halbleitermaterials (4) eingekoppelt wird, wobei kein Sammler zum Auffangen von Fotoelektronen vorhanden ist.

12. Vorrichtung nach Anspruch 11, **dadurch gekennzeichnet, daß** die UV-Strahlung eine Wellenlänge im Bereich von 280 bis 400 nm, insbesondere im Bereich von 320 bis 380 nm aufweist.

## Claims

1. Device with at least one surface layer (1) with an inner side (15), lying on a light conducting carrier (3) and with an outer side (2) as well as with a UV-radiation source (6), wherein the surface layer (1) is formed from a semiconductor material (4) and the UV-radiation (13) from the UV-radiation source (6) is coupled directly into the inner side (15) of the semiconductor material (4) via the light conducting carrier (3) and in this case the outer side (2) of the surface layer (1) is disinfected and/or has oxidising properties, the device having no collector to pick up photo electrons.

2. Device according to claim 1, **characterised in that** the carrier (3) is formed as a plate, which is coated on one side or both sides with the semiconductor material (4).

3. Device according to claim 1, **characterised in that** the carrier (3) is formed as a tube and the semiconductor material (4) is applied to the tube inner area and/or the tube outer area.

4. Device according to claim 1, **characterised in that** the carrier (3) is formed as a container, the inner area of which is coated with the semiconductor material (4).

5. Device according to one of claims 1 to 4, **characterised in that** the carrier material is quartz or a light conducting polymer, in particular polymethylmethacrylate.

6. Device according to one of claims 1 to 5, **characterised in that** the carrier is ductile or flexibly malleable.

7. Device according to one of claims 1 to 6, **characterised in that** the semiconductor material (4) is formed as a thin layer, in particular of a thickness in the order of a few molecules.

8. Device according to one of claims 1 to 7, **characterised in that** the semiconductor material (4) is titanium dioxide or silicon carbide.

9. Device according to one of claims 1 to 8, **characterised in that** a fluorescent dye is incorporated in the semiconductor material (4) and/or in the UV-light source (6) and/or in the light conducting carrier (3).

10. Device according to one of claims 1 to 9, **characterised in that** in addition to the UV-light source (6) an excitation device (10) is provided which electrically excites the semiconductor material (4).

11. Method for disinfecting and/or oxidising on the outer side of a surface layer (1) consisting of a semiconductor material (4), the inner side (15) lying on a carrier (3) using a UV-radiation source (6), **characterised in that** UV-radiation (13) from the UV-radiation source (6) is directly coupled through light cable into the inner side (15) of the semiconductor material (4) via the carrier (3), no collector being provided to pick up photo electrons.

12. Method according to claim 11, **characterised in that** the UV-radiation has a wavelength in the range of 280 to 400 nm, notably in the range of 320 to 380 nm.

## Revendications

1. Appareil avec au moins une couche de surface (1) avec un côté interne (15) qui repose sur un support conducteur de lumière (3) et avec un côté externe (2) ainsi qu'avec une source de rayonnement UV (6), où la couche de surface (1) est formée d'un matériau semiconducteur (4), le rayonnement UV (13) issu de la source de rayonnement UV (6) est couplé sur le support conducteur de lumière (3) directement au côté interne (15) du matériau semiconducteur (4), le côté externe (2) de la couche de surface (1) est alors désinfecté et/ou agit en oxydant, où l'appareil ne comporte aucun collecteur pour capter des photoélectrons.

2. Appareil selon la revendication 1, **caractérisé en ce que** le support (3) est en forme de plaque recouverte d'un côté ou des deux par une couche du matériau semiconducteur (4).

3. Appareil selon la revendication 1, **caractérisé en ce que** le support (3) est en forme tubulaire et le matériau semiconducteur (4) est rapporté sur la surface interne et/ou la surface externe du tube.

4. Appareil selon la revendication 1, **caractérisé en ce que** le support (3) est en forme de récipient dont la surface interne est recouverte par une couche du matériau semiconducteur (4).

5. Appareil selon l'une des revendications 1 à 4, **caractérisé en ce que** le matériau support est du quartz ou un matériau polymère conducteur de lumière, en particulier le polyméthacrylate de méthyle.

6. Appareil selon l'une des revendications 1 à 5, **caractérisé en ce que** le matériau support est plastiquement ou élastiquement déformable.

7. Appareil selon l'une des revendications 1 à 4, **caractérisé en ce que** le matériau semiconducteur (4) est formé d'une couche mince, en particulier avec une épaisseur de couche dont l'ordre de grandeur est de quelques molécules.

8. Appareil selon l'une des revendications 1 à 7, **caractérisé en ce que** le matériau semiconducteur (4) est du dioxyde de titane ou du carbure de silicium.

9. Appareil selon l'une des revendications 1 à 8, **caractérisé en ce qu'**un colorant fluorescent est incorporé au matériau semiconducteur (4) et/ou à la source lumineuse UV (6) et/ou au support conducteur de lumière (3).

10. Appareil selon l'une des revendications 1 à 9, **caractérisé en ce qu'**il est prévu en complément de la source lumineuse UV (6) un dispositif d'excitation (10) qui excite électriquement le matériau semiconducteur (4).

11. Procédé de désinfection et/ou d'oxydation sur le côté externe d'une couche de surface (1) d'un matériau semiconducteur (4) qui repose par son côté interne (15) sur un support (3), par utilisation d'une source de rayonnement UV (6), **caractérisé en ce que** le rayonnement UV (13) issu de la source de rayonnement UV (6) est couplé, par conduction lumineuse, sur le support (3) directement au côté interne (15) du matériau semiconducteur (4), où il n'est prévu aucun collecteur pour capter des photoélectrons.

12. Procédé selon la revendication 11, **caractérisé en ce que** le rayonnement UV présente une longueur d'onde dans la plage allant de 280 à 400 nm, en particulier dans la plage allant de 320 à 380 nm.
